Europäisches Patentamt

(19) European Patent Office

Office européen des brevets

(11) Numéro de publication: **0 220 988**
**B1**

## (12) FASCICULE DE BREVET EUROPEEN

(45) Date de publication du fascicule du brevet:
30.05.90

(21) Numéro de dépôt: 86402315.5

(22) Date de dépôt: 16.10.86

(51) Int. Cl.⁵: **A61K 39/018**

---

(54) Procédé de culture de Babesia canis, application à la préparation d'antigènes et de vaccins, et antigènes et vaccins contre la piroplasmose.

---

(30) Priorité: 24.10.85 FR 8515815

(43) Date de publication de la demande:
06.05.87 Bulletin 87/19

(45) Mention de la délivrance du brevet:
30.05.90 Bulletin 90/22

(84) Etats contractants désignés:
BE CH DE ES GB IT LI NL SE

(56) Documents cités:
EP-A- 0 018 579
EP-A- 0 018 580

BIOLOGICAL ABSTRACTS/RRM, vol. 26, réf.
no. 26052011, 1982; N. LAURENT et al.: "A vaccine for
canine babesiasis using cell culture derived soluble
babesia-canis antigen", & PROCEEDINGS OF THE 5TH
INTERNATIONAL CONGRESS OF PARASITOLOGY,
TORONTO, CA, 7-14 août 1982. MOL. BIOCHEM
PARASITOL 1982, vol. 0, no. suppl. pages 212-213 000
BIOLOGICAL ABSTRACTS, vol. 75, 1983, ref.
no. 10973, P.A., US; E. MOLINAR et al.: "Antigenic and
immunogenic studies on cell culture-derived Babesia
canis", & VET PARASITOL 10(1): 29-40, 1982

(73) Titulaire: RHôNE MERIEUX, 17, rue Bourgelat,
F-69002 Lyon(FR)

(72) Inventeur: Laurent, Nathalie, 2 rue de Boyer,
F-69160 Tassin La Demi-Lune(FR)
Inventeur: Moreau, Yves, Chemin de Bellevue,
F-69260 Marcy l'Etoile(FR)

(74) Mandataire: Bernasconi, Jean et al, CABINET LEMOINE
ET BERNASCONI 13, Boulevard des Batignolles,
F-75008 Paris(FR)

ACTORUM AG

## Description

La présente invention a trait à un procédé de culture de parasites de l'espèce Babesia canis. Elle a également trait à l'application de ce procédé à la préparation d'antigènes et de vaccins contre les Babesioses canines, ainsi qu'aux antigènes et aux vaccins obtenus.

La préparation de vaccins contre les Babesioses a longtemps posé des problèmes pratiquement insurmontables. Ces difficultés étaient liées à l'impossibilité d'obtenir une multiplication industrielle des parasites in vitro ainsi qu'aux difficultés de détecter et d'isoler des antigènes utiles, et notamment des antigènes protecteurs.

Un procédé de multiplication de Babesia a été proposé dans Erp, et al. Am. Jour Trop. Med. Hva., 27 (5) : pp. 1061-1064 (1978). Ce procédé consistait en une propagation des parasites dans une culture d'érythrocytes sous agitation mécanique et avec une teneur accrue de gaz carbonique. Ce procédé n'a cependant pas permis d'atteindre une production de masse.

Les tentatives qui ont été faites, d'autre part, pour produire les parasites de la Babesiose selon des techniques proches de la culture de Plasmodium, décrites dans Trager, et al., Science, 193 : pp. 673-675 (1976) ou Speer, et al., Z. Parasitenk, 50 : pp. 237-244 (1976), n'ont pas abouti.

Plus récemment un procédé a été proposé par Miodrag Ristic et Michael G. Levy, dans le brevet européen n° 18580, pour une production à grande échelle de ces parasites. Ce procédé est basé sur l'incubation dans des cellules érythrocytaires, dans un milieu de culture convenable pour les érythrocytes, auquel est adjoint de 30 à 50 % de sérum, cette incubation s'effectuant de façon statique en présence d'une atmosphère contrôlée contenant de 3 à 6 % de gaz carbonique et dans des conditions permettant de maintenir l'hémoglobine des érythrocytes à l'état désoxydé, à une température de l'ordre de 35 à 38° C. Ce procédé s'est avéré efficace pour la production à grande échelle de Babesia bovis, mais non pas de Babesia canis.

Il est apparu intéressant de pouvoir produire, à grande échelle, Babesia canis dans des conditions pratiques et économiques afin de pouvoir fabriquer un vaccin contre la piroplasmose canine.

L'invention se propose donc de fournir un tel procédé qui permette de façon simple et peu coûteuse de produire à grande échelle le parasite Babesia canis.

Un autre objectif de l'invention est de fournir un tel procédé permettant de préparer des antigènes ou vaccins à partir des surnageants de culture.

Un autre objectif de l'invention est de fournir un procédé d'application du procédé de culture selon l'invention à la préparation d'antigènes ou de vaccins.

Un autre objectif encore de l'invention est de fournir des antigènes, purifiés ou non, et des vaccins, purifiés ou non, à partir des surnageants de culture.

L'invention a pour objet un procédé de culture de parasites de l'espèce Babesia canis, dans lequel on incube des érythrocytes infectés par le parasite dans un milieu de culture convenable pour les érythrocytes, en présence de sérum homologue, à l'état sensiblement statique, caractérisé en ce que l'on met la culture à incuber en atmosphère normale.

Par atmosphère normale dans le sens de la présente invention, on entend une atmosphère composée essentiellement d'oxygène et d'azote et dépourvue de teneur importante en gaz carbonique.

Dans un mode de mise en oeuvre particulièrement avantageux de l'invention, la culture est mise à incuber d'abord à température élevée, notamment entre 34 et 38° C, puis à basse température, notamment entre 0 et 10° C.

De préférence, l'incubation à température élevée est de l'ordre de 8 heures et la durée de culture à basse température de l'ordre de 16 heures. On peut cependant augmenter le temps d'incubation à température élevée et réduire la durée pendant laquelle la culture se trouve à basse température, cette dernière durée n'étant cependant, de préférence, pas inférieure à 6 ou 8 heures.

A la fin de la période d'incubation à basse température, on décante et recueille le milieu surnageant et on remplace celui-ci par du milieu neuf, après quoi on remet la culture à incuber à température élevée, puis à température abaissée et l'on ajuste, de temps en temps, l'hématocrite.

De façon avantageuse, la concentration en érythrocyte, c'est-à-dire l'hématocrite, est de l'ordre de 8 à 12 %, et de préférence de 10 %.

Egalement de façon avantageuse, le milieu de culture comporte entre 20 % et 3 %, et de préférence de l'ordre de 5 % de sérum de chien.

Le milieu de culture préféré est le milieu Ham's F10 ou F12 contenant un peu de glucose et convenablement tamponné. Ainsi le milieu peut par exemple comprendre du glucose à 1g/l, un tampon Hepes et du bicarbonate à environ 1,2 % final.

Cependant d'autres milieux usuels peuvent être utilisés, tels que par exemple, 199 avec sels de Hanks ou RPMI 1640. Les milieux de culture doivent être compatibles avec la survie des érythrocytes et contenir les éléments permettant la multiplication des parasites. Ceci peut être obtenu en partant de milieux de culture riches et en faisant des essais convenables.

Dans le cas où le procédé de culture est destiné à produire des substances destinées à être administrées à l'animal, par exemple un vaccin, la culture s'effectue avantageusement en présence d'un antibiotique tel que par exemple la gentamycine.

La culture est effectuée à un pH convenable pour le développement des parasites, ce pH étant de l'ordre de 7,3 à 7,5 et de préférence de l'ordre de 7,4.

Conformément à l'invention, le surnageant de culture est recueilli à la fin de la période d'incubation à basse température. Ce surnageant peut être utilisé soit directement, soit après des étapes de purification, pour former des antigènes et/ou un vaccin contre la piroplasmose canine.

De préférence, on recueille le surnageant lorsque la concentration en parasites atteint au moins $10^8$ parasites/ml.

De façon avantageuse, le surnageant est soumis à une centrifugation de façon à éliminer les cellules, les parasites et les débris cellulaires et débris de parasites, après quoi on procède à une filtration stérilisante. Le surnageant peut ensuite être avantageusement concentré, par exemple par ultrafiltration.

Pour la préparation d'un vaccin, on peut ensuite avantageusement inactiver les contaminants éventuels du surnageant par exemple au formol ou la bétapropiolactone ou éthyl-éthylénimine.

Le vaccin se présente de préférence sous forme lyophilisée.

Le vaccin peut être administré par voie sous-cutanée.

Un vaccin contre la piroplasmose, selon l'invention, est caractérisé en ce qu'il comporte des antigènes solubles et concentrés de Babesia canis en provenance d'un surnageant de culture de Babesia canis sur érythrocyte.

De préférence, la dose contient au maximum 0,120 mg de formol ou 20 % de bétapropiolactone ou d'éthyl-éthylénimine.

Le solvant du vaccin lyophilisé comporte de préférence un adjuvant et se présente, par exemple, sous forme d'une solution de saponine, par exemple à 0,5 mg/ml.

D'autres avantages et caractéristiques de l'invention apparaîtront à la lecture de la description suivante, faite à titre d'exemple non limitatif.

Exemple 1 : <u>culture de parasite</u>

On recueille le sang d'un chien splénectomisé infecté expérimentalement, par voie intraveineuse, à l'aide d'une souche de Babesia canis congelée en azote liquide. Le sang parasité est récolté par ponction intracardiaque, sur héparine, à la concentration finale de 100 U/ml. Le plasma et les leucocytes sont éliminés par une première centrifugation à 400 g pendant 10 minutes. Le culot de globules rouges est ensuite lavé deux fois en tampon PBS 0,15 M, pH 7,2 à 400 g pendant 10 minutes.

On réalise alors une suspension du culot de globules rouges à la concentration de 10 % dans un milieu de culture constitué de 5 % de sérum de chien et de 95 % de milieu Ham's F10 contenant du glucose à 1 g/l, un tampon Hépes à 25 mM, du bicarbonate à 1,2 % et de la gentamycine à 100 mg/l. Le pH de la suspension de globules rouges parasités est ajusté à 7,4 avec de la soude 1M.

La suspension est ensuite répartie dans des flacons de culture cellulaires à raison de 100 ml pour 150 cm².

Les flacons sont ensuite mis à incuber dans une étuve en atmosphère normale humide à une température de + 37° C pendant une durée de 8 heures. Ensuite les flacons sont amenés à une température de + 4° C pendant 16 heures.

A la fin de cette incubation à + 4° C, le milieu surnageant des flacons est décanté et recueilli. Ce milieu est remplacé dans les flacons par du milieu neuf et les cultures sont remises ensuite à incuber pendant 8 heures à 37° C.

Le cycle de culture se poursuit et, toutes les 72 heures, des érythrocytes sains sont ajoutés à la culture de façon à rajuster l'hématocrite à 10 % par rapport au milieu.

Le surnageant recueilli, dépourvu de globules rouges et de parasites, contient les antigènes recherchés.

Exemple 2 : <u>préparation d'un vaccin contre la piroplasmose</u>

Les surnageants recueillis à la fin de plusieurs incubations successives sont mélangés. Le mélange de surnageants ainsi obtenu est soumis à une centrifugation à 700 g destinée à éliminer les globules rouges et les éventuels parasites ainsi que leurs débris. La durée de la centrifugation est de 15 mn.

Le surnageant ainsi centrifugé est ensuite soumis à une filtration stérilisante sur membrane 0,22 µ.

Le surnageant est ensuite soumis à une concentration par ultra-filtration sur une membrane 20 000 Daltons.

Le concentrat est traité au formol à une teneur de 0,12 mg/ml pendant une nuit à une température de + 4° C.

Il est ensuite lyophilisé et réparti en doses correspondant chacune à au moins $10^8$ parasites/ml.

Pour l'administration aux chiens, le vaccin lyophilisé est dissous dans une solution de saponine à 0,5 mg/ml dans de l'eau stérile apyrogène.

Le vaccin est administré par voie sous-cutanée, de préférence dans la région sous-scapulaire, à raison d'une dose. La primo-vaccination comporte deux injections à trois-quatre semaines d'intervalle. On effectue des rappels annuels ou semestriels.

La quantité de solvant à la saponine est adaptée au poids du chien à vacciner, les chiens d'un poids supérieur à 7 kilos recevant la dose dissoute dans 1 ml de solvant et les chiens d'un poids inférieur à 1 kilo recevant la dose dissoute dans 0,5 ml de solvant.

Bien que l'invention ait été décrite à propos d'une forme de réalisation particulière, il est bien entendu qu'on peut lui apporter diverses modifications de détails sans pour cela s'éloigner ni de son cadre ni de son esprit.

**Revendications**

1. Procédé de culture de parasites de l'espèce Babesia canis, notamment pour la préparation d'antigènes ou de vaccins, dans lequel on incube des érythrocytes infectés par le parasite dans un milieu de culture convenable pour la survie des érythrocytes et la multiplication des parasites, en présence de sérum homologue, notamment à l'état statique, caractérisé en ce que l'on met la culture à incuber en atmosphère normale, à savoir composée essentiellement d'oxygène et d'azote et dépourvue de teneur importante en gaz carbonique.

2. Procédé selon la revendication 1, caractérisé en ce que l'on met la culture à incuber d'abord à température élevée, notamment entre 34 et 38°C, puis à basse température, notamment entre 0 et 10°C.

3. Procédé selon la revendication 2, caractérisé en ce que l'incubation à température élevée est de 8 heures et l'incubation à basse température est de 16 heures.

4. Procédé selon l'une quelconque des revendications 1 à 3, caractérisé en ce qu'en fin de culture à basse température, on décante et recueille le milieu surnageant et on le remplace par du milieu neuf, après quoi on remet la culture à incuber à température élevée, puis à température abaissée et on ajuste, si nécessaire, l'hématocrite.

5. Procédé selon l'une quelconque des revendications 1 à 4, caractérisé en ce que l'hématocrite est de 8 à 12% et notamment 10%.

6. Procédé selon l'une quelconque des revendications 1 à 5, caractérisé en ce que le milieu de culture contient entre 20% et 3%, et notamment de 5%, de sérum de chien.

7. Procédé selon l'une quelconque des revendications 1 à 6, caractérisé en ce que le milieu de culture est à base de milieu Ham's F10 ou F12, ou 199 avec sels de Hanks ou RPMI 1640, contenant un peu de glucose et convenablement tamponné.

8. Procédé selon la revendication 7, caractérisé en ce que le milieu comprend du glucose à 1 g/l, un tampon Hépes et du bicarbonate à environ 1,2%.

9. Procédé selon l'une quelconque des revendications précédentes, caractérisé en ce que la culture est effectuée à un pH de 7,3 à 7,5 et notamment de 7,4.

10. Procédé selon l'une quelconque des revendications 1 à 9, caractérisé en ce que l'on effectue la culture en présence d'un antibiotique et notamment la gentamycine.

11. Procédé selon l'une quelconque des revendications 1 à 10, caractérisé en ce que l'on recueille l'inoculum de parasites sur héparine.

12. Procédé selon l'une quelconque des revendications 1 à 11, caractérisé en ce que l'on soumet le surnageant de la culture à un centrifugation puis à une filtration stérilisante.

13. Procédé selon la revendication 12, caractérisé en ce que l'on concentre le surnageant.

14. Procédé selon l'une des revendications 12 et 13, caractérisé en ce que l'on inactive les contaminants éventuels du surnageant et qu'on le lyophilise.

## Claims

1. A method for the culture of parasites of the Babesia canis species, in particular for the preparation of antigenes or vaccines, in which erythrocytes infected by the parasite are incubated in a culture medium suitable for the survival of the erythrocytes and for breeding of the parasites, in the presence of a homologous serum, in particular in the static state, characterized in that the culture is placed to incubate in a normal atmosphere, i.e. in an atmosphere composed essentially of oxygen and nitrogen and devoid of any substantial carbonic gas content.

2. The method as claimed in claim 1, characterized in that the culture is placed first of all in a high temperature, more especially between 34 and 38°C, to incubate, then in a low temperature, more especially between 0 and 10°C.

3. The method as claimed in claim 2, characterized in that the high temperature incubation lasts for 8 hours and the low temperature incubation for 16 hours.

4. The method as claimed in any one of claims 1 to 3, characterized in that at the end of the low temperature culture, the supernatant medium is decanted and collected and it is replaced by new medium, after which the culture is replaced in a high temperature to be incubated, then at a lower temperature and if required the hematocrit is adjusted.

5. The method as claimed in any one of claims 1 to 4, characterized in that the hematocrit is of the order of 8 to 12% and more especially 10%.

6. The method as claimed in any one of claims 1 to 5, characterized in that the culture medium comprises between 20% and 3% and more especially of the order of 5% of dog serum.

7. The method as claimed in any one of claims 1 to 6, characterized in that the culture medium has a Ham's medium F10 of F12, or 199 with Hanks salts of RPMI 1640 base containing a little glucose and suitably buffered.

8. The method as claimed in claim 7, characterized in that the medium comprises glucose at 1 g/l, a Hepes buffer and bicarbonate at about 1.2%.

9. The method as claimed in any one of the preceding claims, characterized in that the culture is carried out at a pH of the order of 7.3 to 7.5 and more especially of the order of 7.4.

10. The method as claimed in any one of claims 1 to 9, characterized in that the culture is carried out in the presence of an antibiotic and more especially gentamycine.

11. The method as claimed in any one of claims 1 to 10, characterized in that the parasite inoculum is collected on heparine.

12. The method as claimed in any one of claims 1 to 11, characterized in that the supernatant culture product is subjected to centrifugation then to sterilizing filtration.

13. The method as claimed in claim 12, characterized in that the supernatant product is concentrated.

14. The method as claimed in any one of claims 12 and 13, characterized in that the possible contaminants of the supernatant product are inactivated and the latter is lyophilized.

## Patentansprüche

1. Verfahren zur Kultur von Parasiten der Spezies Babesia canis, insbesondere zur Herstellung von Antigenen oder Impfstoffen, bei welchem man die mit dem Parasiten infizierten Erythozyten in einem Nährmedium, welches für das Überleben der Erythrozyten und die Vermehrung der Parasiten geeignet ist, in Gegenwart des Serumhomologen, insbesondere im statischen Zustand, inkubiert, dadurch gekennzeichnet, daß man die Kultur in normaler Atmosphäre, nämlich zusammengesetzt im wesentlichen aus Sauerstoff und Stickstoff und ohne wesentlichen Gehalt an Kohlensäuregas, inkubiert.

2. Verfahren nach Anspruch 1, dadurch gekennzeichnet, daß man die Kultur zuerst bei einer erhöhten Temperatur, insbesondere zwischen 34°C und 38°C, und anschließend bei einer niedrigen Temperatur, insbesondere zwischen 0°C und 10°C, inkubiert.

3. Verfahren nach Anspruch 2, dadurch gekennzeichnet, daß die Inkubation bei der erhöhten Temperatur acht Stunden und die Inkubation bei der niedrigen Temperatur 16 Stunden beträgt.

4. Verfahren nach einem der Ansprüche 1 bis 3, dadurch gekennzeichnet, daß man, bei Beendigung der Kultur bei einer niedrigen Temperatur, den Kulturmediumüberstand sammelt und durch ein neues Medium ersetzt, worauf man die Kultur wieder bei der erhöhten Temperatur, dann bei der niedrigen Temperatur inkubiert, und, falls notwendig, den Hematokriten einstellt.

5. Verfahren nach einem der Ansprüche 1 bis 4, dadurch gekennzeichnet, daß der Hematokrit 8% bis 12% und insbesondere 10% beträgt.

6. Verfahren nach einem der Ansprüche 1 bis 5, dadurch gekennzeichnet, daß das Kulturmedium zwischen 20% und 3% und insbesondere 5% Hundeserum enthält.

7. Verfahren nach einem der Ansprüche 1 bis 6, dadurch gekennzeichnet, daß das Kulturmedium als Mediumgrundlage Ham's F10 oder F12 oder 199 mit Hanks Salzen oder RPMI 1640 aufweist und etwas Glukose und einen geeigneten Puffer enthält.

8. Verfahren nach Anspruch 7, dadurch gekennzeichnet, daß das Medium 1 g/l Glukose, einen Hepes-Puffer und Bicarbonat von etwa 1,2% enthält.

9. Verfahren nach einem der vorhergehenden Ansprüche, dadurch gekennzeichnet, daß die Kultur bei einem pH-Wert von 7,3 bis 7,5 und insbesondere von 7,4 durchgeführt wird.

10. Verfahren nach einem der Ansprüche 1 bis 9, dadurch gekennzeichnet, daß die Kultur in Gegenwart eines Antibiotikums und insbesondere von Gentamycin durchgeführt wird.

11. Verfahren nach einem der Ansprüche 1 bis 10, dadurch gekennzeichnet, daß man das Parasiteninokulum über Heparin sammelt.

12. Verfahren nach einem der Ansprüche 1 bis 11, dadurch gekennzeichnet, daß man den Kulturüberstand einer Zentrifugation und anschließend einer Sterilfiltration unterwirft.

13. Verfahren nach Anspruch 12, dadurch gekennzeichnet, daß man den Überstand konzentriert.

14. Verfahren nach einem der Ansprüche 12 und 13, dadurch gekennzeichnet, daß man etwaige Verunreinigungen des Überstandes inaktiviert und daß man ihn lyophilisiert.